# EUROPEAN PATENT SPECIFICATION

(11) **EP 3 057 501 B1**
(45) Date of publication and mention of the grant of the patent: **26.12.2018**
(21) Application number: 14825435.2
(22) Date of filing: 17.10.2014
(51) Int. Cl.: A61B 5/0408, A61B 5/00

(54) **MEDICAL DEVICE FOR THE DETECTION, MEASUREMENT, IN AN UNBLOODY AND NON-INVASIVE MANNER, AND TRANSMISSION OF VITAL, BIOMEDICAL PARAMETERS, SUCH AS CARDIOVASCULAR AND RESPIRATORY PARAMETERS, OF A HUMAN BODY**
MEDIZINISCHE VORRICHTUNG ZUR UNBLUTIGEN UND NICHTINVASIVEN DETEKTION, MESSUNG UND ÜBERTRAGUNG BIOMEDIZINISCHER VITALZEICHEN, WIE Z. B. KARDIOVASKULÄRER UND ATEMPARAMETER, EINES MENSCHLICHEN KÖRPERS
DISPOSITIF MÉDICAL POUR LA DÉTECTION, LA MESURE, DE FAÇON NON-SANGLANTE ET NON-INVASIVE, ET LA TRANSMISSION DE PARAMÈTRES BIOMÉDICAUX VITAUX, TELS QUE DES PARAMÈTRES CARDIOVASCULAIRES ET RESPIRATOIRES, D'UN CORPS HUMAIN

(30) Priority: 18.10.2013 IT MI20131745
(43) Date of publication of application: 24.08.2016
(73) Proprietor: Salerno, Mario, 95125 Catania (IT)
(72) Inventor: Salerno, Mario, 95125 Catania (IT)
(74) Representative: Baroni, Matteo
(86) International application number: PCT/IB2014/065414
(87) International publication number: WO 2015/056239

(56) References cited:
- JP-A- 2007 202 939
- US-A- 4 715 367
- US-A1- 2007 142 738
- US-A1- 2008 184 574
- US-A1- 2013 046 181

## Description

### Application field of the invention

The present invention refers to a medical device for detecting, measuring, and transmitting, in a non-invasive, continuous and instantaneous way, vital parameters of the human body, and other biomedical parameters, and consequent operations, especially for indicating the need of an intervention.

### Description of the prior art

WHO (World Health Organization) defines health as "A state of complete physical, mental and social well-being and not merely the absence of disease or infirmity". Such definition defines the fundamental right to health.

Therefore a broader and broader demand is increasingly developing, not only for treating the different diseases, but above all for:
- preventing the illnesses due to the increase of the average life expectancy;
- intervening in an early, prompt and adequate way for those acute diseases, above all cardiovascular and respiratory, which are a potential cause of death and which may provoke disabilities;
- controlling and monitoring vital parameters and clinical data in life-threatening chronic illnesses and/or to prevent the development of secondary handicaps.

In parallel to such a pressing demand, in all domestic economies the need for coherent and documented diagnosis arises, in order to give a justified access to both hospitalization and sophisticated, and therefore expensive, medical examinations and procedures, and thus avoid wasting precious economical resources.

Currently, by activating the different medical diagnostic devices, both in acute and in chronic diseases, it is not possible to obtain an immediate and general evaluation of those parameters, that in some patients are crucial to define the diagnosis and start a treatment, above all in the most urgent cases.

It is important to have access, above all, to fundamental detections:
- in patients with chronic cardiovascular, respiratory diseases and in their emergencies due to relapse or complications;
- in healthy subjects which have an acute symptomatology referring to the cardiovascular and/or respiratory apparatus;
- in patients having chronic diseases for which some vital parameters need to be monitored. Currently, medical devices that arc able to provide and, at the same time, to send data in an instantaneous or continuous non-invasive way, in order to respond to such medical and diagnostic needs, for both acute and chronic cardiovascular and respiratory diseases, arc not known in the art.

The known devices, such as for example the ones described in US2007/142738-A1 or in US2006/184059-A1, allow to record and to send heartbeat, and to record electromyography graphs in a non-invasive way, to evaluation centres.

In recent times, modern smartphones and tablets, which are actually starting to include pre-installed medical software applications, are also used for both receiving and transmitting analysis and responses to detection centres, also as a support of amateur and agonistic sport activities.

US2013/014706-A1 describes a collar-type device for veterinary applications to animals, which comprises some sensors adapted to measure the animal's parameters and to transmit them, also remotely. The device comprises an inflatable band adapted to create a tension of the collar against the neck of the animal, so as to keep it still and adherent in order to press the sensors against the neck of the animal itself. In addition to the functional limitations of such device, and to the dangerousness of pressing a collar against the neck of a person, it is not adapted to applications to the human body, as it is not possible to obtain reliable detections for critical parameters, nor for other complex parameters that are useful and necessary in the cardiovascular and respiratory emergencies.

Currently, medical devices adapted to the use on human body that have all the following requirements arc not known in the art:
- ability to detect, record and transmit vital parameters and complex biomedical parameters to the home or to the hospital;
- ability to carry out immediate or remote operations for cardiovascular emergencies, using a single or multiple parameters;
- easy application to patient;
- ability to store the parameters detected, both wired and wireless interactivity on smartphones, PCs and networks;
- ability to localize and identify the device and thus the patient;
- flexibility to implement the connection of detectors of other parameters;
- non-invasiveness;
- simple management.

### Summary of the invention

Therefore the aim of the present invention is to provide a medical device adapted to detect, to measure, in a bloodless and non-invasive way, and to transmit vital, biomedical parameters, such as cardiovascular and respiratory ones, of the human body, able to overcome all the aforementioned drawbacks.

It is an object of the present invention a medical device adapted to detect, to measure, in a bloodless and non-invasive way, and to transmit vital, biomedical parameters, such as cardiovascular and respiratory ones, of the human body.

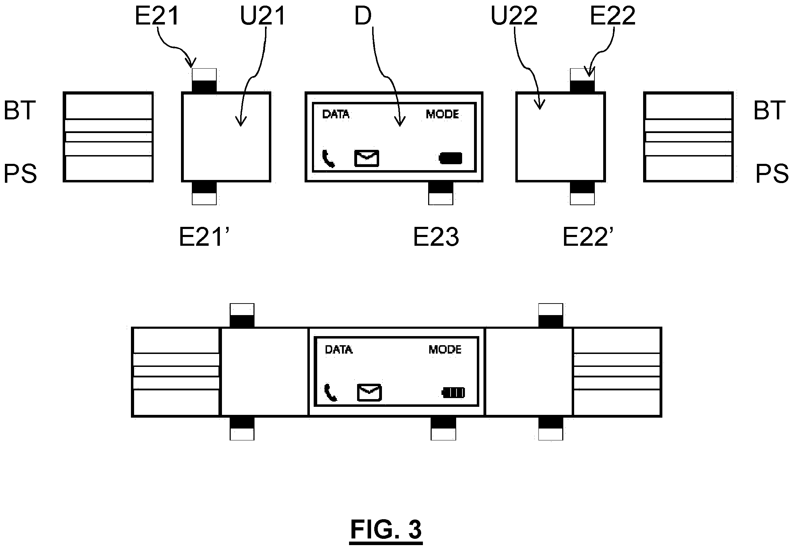

The invention is defined by claim 1. Figures 3, 4 and 5 disclose an embodiment of the invention (second embodiment of the disclosure). The first embodiment of the disclosure is an exemplary embodiment not forming part of the invention.

It is particular object of the present invention a medical device adapted to detect, to measure, in a non-invasive way, and to transmit vital, biomedical parameters, such as cardiovascular and respiratory ones, of the human body, as described more fully in the claims that arc considered as an integral part of the present description.

### Brief description of the figures

Further aims and advantages of the present invention will become more apparent from the following detailed description of an embodiment thereof (and or its alternative embodiments) and from the annexed drawings, which are supplied by way of non-limiting example, wherein:
figure 1 shows a first embodiment of the device that is οbject of the present invention;
figure 2 shows the device of fig. 1 extended in a straight line, with indication of the elements connected to it;
figure 3 and 4 show a second embodiment of the device that is object of the present invention, with units that can be assembled separately or that arc assembled on one single support;
figure 5 shows an example of application of the device for measuring medical parameters in some body areas;
figures 6.1, 6.2 show two possible embodiments of the device.

### Detailed description of embodiments

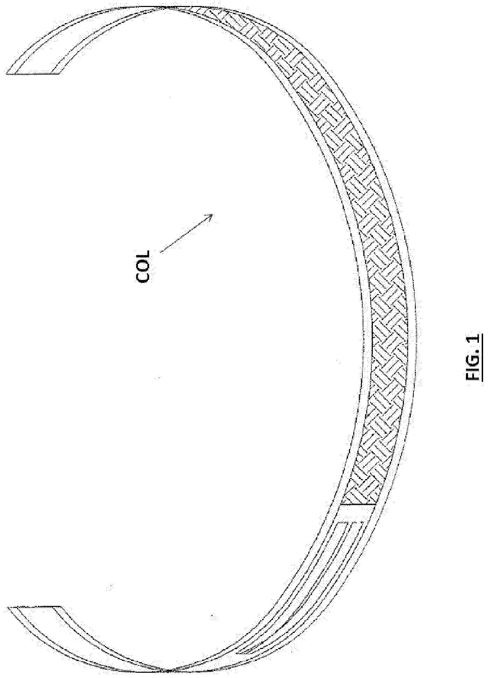

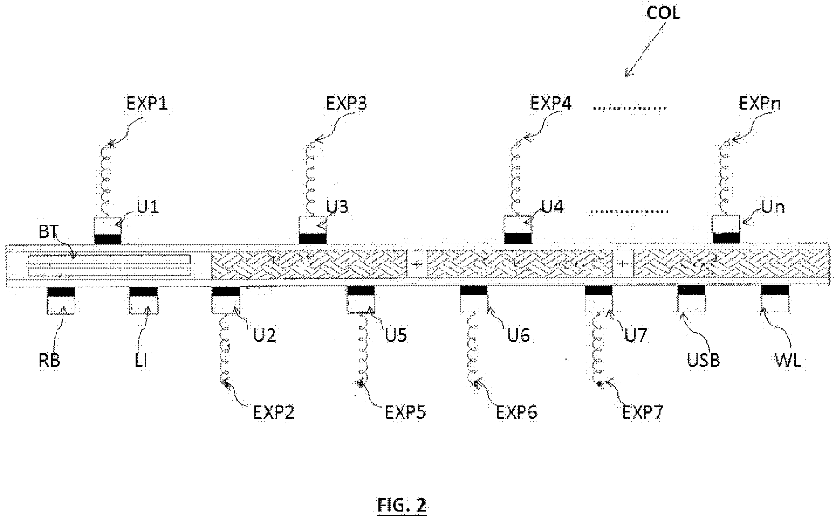

In a first embodiment, with reference to figures 1 and 2, the device is shaped, by way of example, as a collar COL formed by a strip-shaped flexible support made of suitable material, an appropriate fastening device (not shown), for example a clip or a button, being placed at its ends.

A number of units U1, U2, ..... made in a way per se known and able to detect body parameters are connected to the device COL. Each unit is adapted to be connected to one or more relating explorers EXP1, EXP2, ......EXPn either by means of possibly extentable and retractable wires, or in wireless mode.

In addition, further connection interfaces, such as USB, WL (wireless) are connected to the device COL for transmitting and receiving data and parameters towards suitable external units.

The device (COL) further comprises connections for battery recharging (RB), by using modem technology for installing batteries that ensures its safety and durability, possibly using micro solar panels to have another energy source.

For the identification and localization of the patient to which the device is applied, **GPS** satellite localizers are placed on the site **LI.** Also, in such site, a microchip is positioned for the identification of the patient using a PIN or other identification sequence.

Furthermore, in such site LI a SIM card is inserted which provides both the patient localization and identification cither on WEB or on other detection device, protecting his/her privacy.

By adding a SIM card, the device acquires an important smartphone function, with all its current and future operating potential. That, in addition to all of its multiple applications (for example a micro-camera), allows the patient to have a normal cell phone available that connects him/her directly (also using speakerphone or earphones) to other apparatuses or to a help centre, providing immediately his/her position, his/her identification and the possibility of talking directly to the operators.

The device **COL,** of reduced dimensions, may become a smartphone by just inserting the SIM card, freeing the patient's, or any possible user's, hands and being itself either only a smartphone or a device able to send an help request to help centres.

What just said about localization and identification of the patient may and will be improved by new micro-technologies.

The eccentric, namely peripheral, position of the site LI (Localization, Identification) and RB (battery recharging) on the collar allows to avoid any interference with the normal bioelectric activity of the heart.

The units U1, U2, ..... Un and the interfaces USB, WL are connected to suitable hardware and software components integrated in the device, and adapted to receive the data detected by the units, store them, organise them and transmit them, in a not encrypted or encrypted way for privacy reasons, immediately or at suitable times, even on demand, to external units such as PC, tablets, smartphones, being them single or organized in a network, placed at the patients' home or in evaluation centres where data can undergo further processing and integration using hardware and software suitable for the detected data. The latter may, in its turn, offer immediate responses or communicate with patients or medical, paramedical personnel at the patients' home or at a hospital.

The electronic components of the collar COL are battery-powered: the batteries. BT can be integrated inside the collar.

The collar or necklace shape is important as it is anatomical and can be applied in an anatomically suitable area on the human body. Thus the collar or necklace shape is, crucial for making its usage easy and effective; this is because it can be worn fast and easily, as it is located in the proximity of most of the organs of cardiovascular and respiratory interest that is object or the detection of biomedical parameters.

The device COL can be made in a single piece or by articulated meshes, for example of the modular type, each mesh being able to connect a respective unit, so as to combine consecutive units.

The collar can be made of light, anallergie material, made of carbon fibre with a fastener, e.g. having dimensions 3 cm x 80 cm.

The different units U1, U2, ..... Un can be activated only if needed, not necessarily all together. They can be connected externally to one or both most elongated sides of the collar.

The different units are expected to communicate with each other by means of the electronic components of the collar, for example defibrillator and cardiac arrhythmia detector; once the arrhythmia is detected, it is communicated to the defibrillator which is immediately activated.

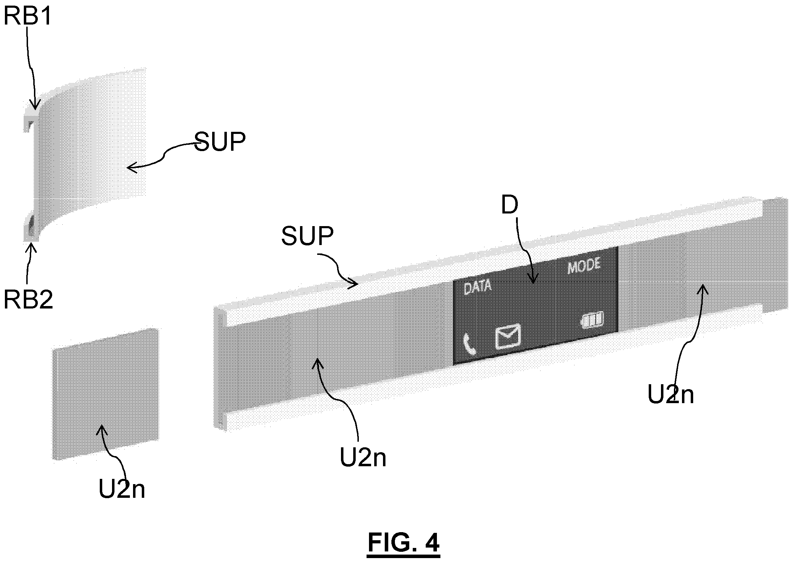

A second embodiment of the medical device is shown in figures 3, 4 e 5.

The device comprises essentially:
- a flexible strip-shaped support SUP, preferably collar or neeklace shaped; it comprises folds RB1, RB2 at the edges of the longer sides so as to make a rail on which the different detection or possibly transmission operating units U21....U2n, or a single case containing the different units U21....U2n, can be inserted in a sliding and removable way. The strip of the flexible support is thus turned towards the inner side of the support towards the body of the person, while its opposite outer side remains open, in order to show most of the surface of the elements accommodated inside the support.
- detection and possibly transmission operating units U21....U2, Un, which can be made by single components which carry out the different functions, or a single case that supports the different operating units.
- exploring probes EXP1, EXP2, EXPn adapted to be connected to said operating units U1....Un by means of connections E21....E23 and possibly extendable and retractable wires or in wireless mode.
- plates placed on the anatomical sites of the person to be analyzed, adapted to connect said explorers and wires to the body. Plates are of a type known in the art, adapted to ensure a long adherence, in an anallergic way, and make the adherence, the data transmission and thus the reading perfect, reducing the recording errors. Said plates may serve as detectors, data integration and transmission in wireless, Bluetooth or other mode, to allow a new type of mobility in the data transmission.
- at least one display D preferably of the touchscreen type. The presence of the touchscreen display may allow the user or the operator, with respective suitable icons, to use immediately all the functions of the device, including all its operations and data storing.
- at least one SIM card with the functions described above with reference to the first embodiment of the device.
- at least one battery BT compartment, preferably of the rechargeable type.
- miniaturized solar panels PS for battery recharging and for increasing the endurance and the mobility of the device.
- USB ports to connect other devices
- wireless, Bluetooth telecommunication devices
- connection (not shown), also of the wireless type, for a keyboard for the input of data and commands.

Other general characteristics can be referred to what described above with reference to the first embodiment of the device.

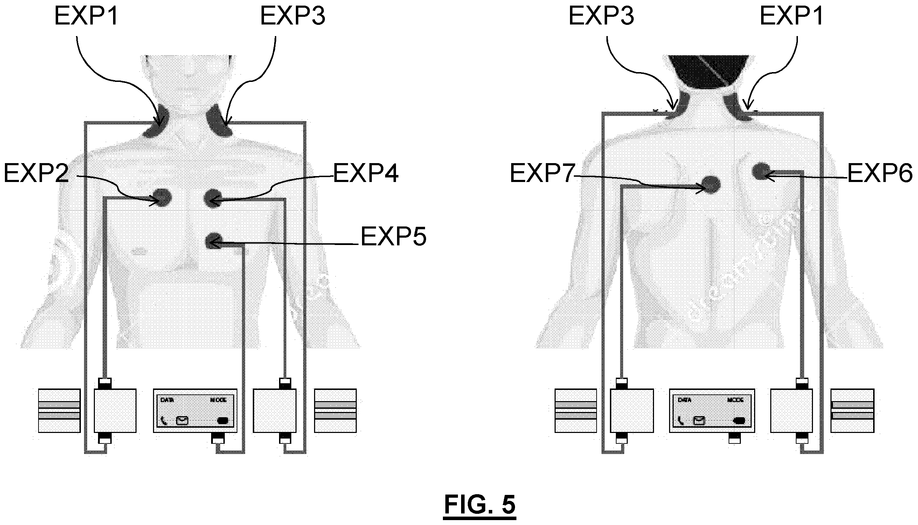

Figure 5 shows examples of anatomical sites, where the mentioned detections and operations take place, such as:
- area of the neck, at the base of the neck
- right and left second parasternal intercostal space
- fifth space on the left midelavicurlar line
- right dorsal scapular and interscapular area.

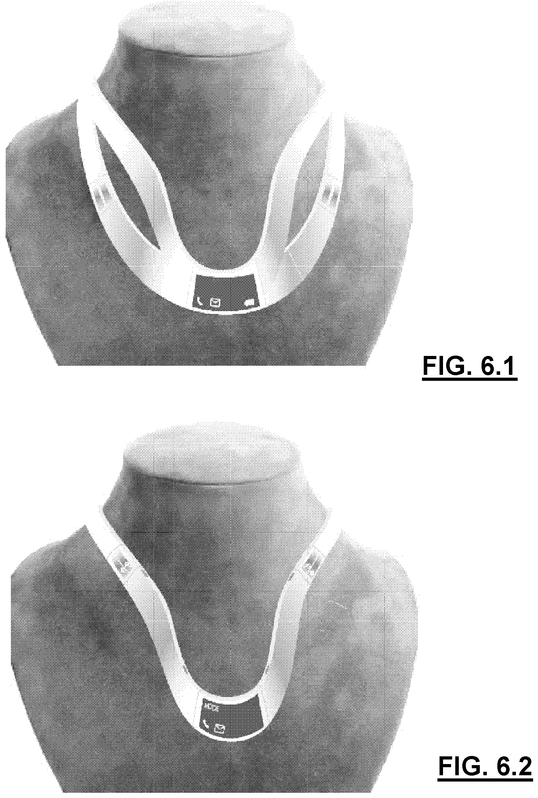

Figures 6.1, 6.2 show two possible embodiments of the device with flexible collar or necklace shaped support, to allow an easy application on the neck area of the person.

The flexible support can be made of plastic or carbon fibre with a fastener, e.g. having dimensions 3 cm x 80 cm.

The particular shape of the device allows, for example:
- any easy application on the neck area of the patient;
- an easy access for the deteclors to the anatomical sites from which they can
   a- display the vital parameters that are object of the detection, namely: skin temperature, arterial pressure, Sp02%; heartbeat;
   b- acquire the Doppler ultrasonography of ascending aorta;
   c- record the ECG (electrocardiography) in a continuous and instantaneous way;
   d- acquire M-mode and B-m echocardiography (parasternal and apical 5 chamber view)
   e- intervene, from an operation point of view, with PM (temporary transcutaneous pacemaker) and cardiac defibrillator.
   f- localize and identify the patient quickly;
   g- make its shape flexible, which can be modified and lightened;
   h- have pre-programmed cut-offs, which can be visual or acoustic, thus limiting the data transmission and providing immediately an alert warning to the user;
   i- reduce improper or incoherent hospitalization having documented the data of the diseases that are object οf the detection;
   l- have fast operating responses with an overall picture documented by the diseases that are object of the data detection;
   m- communicate, also using the smartphone function inserted in the collar, with the patient providing him/her with indications;
   n- let the patient free from the fear to be far from an help centre.
   o- access to the display for setting, desired information and necessary operations.

Indeed, the clinical data acquired by the device, by using detectors placed in well-defined anatomical areas, are not just relating to vital parameters detected by a generie rescuer, by the so-called "primary exam" (arterial pressure, heartbeat, body temperature, consciousness), but also to more complex cardiovascular and respiratory data (electrocardiography, Doppler ultrasonography of carotid axis, percentage of oxygen saturation), that, in acute emergencies, above all cardio-respiratory ones, may provide documented data adapted to provide operating responses at different levels, namely:
- immediately on the patient;
- by activating a complete information that may remotely command the response units inserted in the device (temporary transcutaneous pacemaker; defibrillator);
- by creating an information flow that allow the highest promptness and effectiveness in the medical intervention (ambulance, emergency room, etc.).

The device that is object or the patent is a medical device, in the sense that its application, management, and the interventions that arc considered appropriate arc in charge of specialized medical personnel only. Users may possibly have access to simple detections (skin temperature, arterial pressure, heartbeat), but always under medical supervision.

It will be apparent to the person skilled in the art that other equivalent embodiments, and their combinations, of the invention can be conceived and reduced to practice without departing from the scope of the invention.

The elements and the characteristics shown in the different preferred embodiments can be combined with each other without departing from the scope of the present patent.

From the description set forth above the person skilled in the art is able to realize the object of the invention without introducing further constructive details.

## Claims

1. A medical device adapted to detect, to measure, in a bloodless and non-invasive way, and to transmit vital, biomedical parameters, such as cardiovascular and respiratory ones, of the human body; the device comprises:
- a flexible strip-shaped support (SUP) adapted to be applied on the human body in the area of the neck, and to be operatively closed around the neck, said support being made in a single piece;
- more than one exploring probes (EXP1, EXP2, ......EXPn) external to the support, each probe adapted to measure body parameters;
- more than one units (U1, U2, ..... Un) adapted to receive said body parameters coming from respective one of said exploring probes (EXP1, EXP2, ......EXPn); each unit adapted to be activated independently from the other units and said support being able to connect a respective unit;
- one or more connection interfaces for transmitting and receiving data and said parameters towards external units;
- hardware and software electronic components integrated inside the flexible support and adapted to receive said body parameters from said units (U1, U2, ..... Un), store them, organize them and transmit them as encrypted or not encrypted way by means of said one or more connection interfaces;
- said device being adapted to record an electrocardiography in a continuous or instantaneous way, and/or to acquire the Doppler ultrasound of ascending aorta, and/or to acquire m-B mode echocardiography, and/or to immediately or remotely operate on emergency intervention units such as a temporary transcutaneous pacemaker and/or a cardiac defibrillator, **characterised in that**
- said flexible strip-shaped support (SUP) comprises folds (RB1, RB2) at the edges of the longer sides adapted to make a rail to insert in a sliding and removable way said one or more units (U1, U2, ..... Un), and/or said one or more connection interfaces, and/or said hardware and software electronic components..

2. A medical device adapted to detect, to measure, in a non-invasive way, and to transmit vital, biomedical parameters, such as cardiovascular and respiratory ones, of the human body, as in claim 1, wherein said electronic components are powered by batteries (BT) integrated in the flexible support, said collar comprising connections to recharge the batteries and means for identifying the patient (LI).

3. A medical device adapted to detect, to measure, in a non-invasive way, and to transmit vital, biomedical parameters, such as cardiovascular and respiratory ones, of the human body, as in claim 2, wherein said means for identifying the patient (LI) comprise a GPS satellite localizer and a SIM card, said device being able to serve as a smartphone.

4. A medical device adapted to detect, to measure, in a non-invasive way, and to transmit vital, biomedical parameters, such as cardiovascular and respiratory ones, of the human body, as in claim 1, wherein said strip is turned on the inner side of the support towards the body of the person, while the opposite outer side is open.

5. A medical device adapted to detect, to measure, in a non-invasive way, and to transmit vital, biomedical parameters, such as cardiovascular and respiratory ones, of the human body, as in claim 1, wherein said one or more units (U1, U2, ..... Un) are adapted to communicate with each other by means of said electronic components.

6. A medical device adapted to detect, to measure, in a non-invasive way, and to transmit vital, biomedical parameters, such as cardiovascular and respiratory ones, of the human body, as in any one of the preceding claims, wherein said flexible strip-shaped support is collar or necklace shaped.

7. A medical device adapted to detect, to measure, in a non-invasive way, and to transmit vital, biomedical parameters, such as cardiovascular and respiratory ones, of the human body, as in any one of the preceding claims, wherein the medical device is made of plastic or carbon fibre, adapted to be positioned on the human body in a position that does not interfere with the cardiac bioelectric activity.

8. A medical device adapted to detect, to measure, in a non-invasive way, and to transmit vital, biomedical parameters, such as cardiovascular and respiratory ones, of the human body, as in claim 7, wherein the medical device further comprises a fastener that is placed at the ends of the flexible support.

9. A medical device adapted to detect, to measure, in a non-invasive way, and to transmit vital, biomedical parameters, such as cardiovascular and respiratory ones, of the human body, as in any one of the preceding claims, wherein the medical device comprises a support for smartphone or other transmission means.

10. A medical device adapted to detect, to measure, in a non-invasive way, and to transmit vital, biomedical parameters, such as cardiovascular and respiratory ones, of the human body, as in claim 1, comprising plates adapted to be placed on anatomical sites and connected to said exploring probes (EXP1, EXP2, ......EXPn), said plates being adapted to become a seat for data integration and transmission in wireless, or Bluetooth mode.

## Patentansprüche

1. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen; wobei die Vorrichtung umfasst:
- eine flexible streifenförmige Stütze (SUP), die angepasst ist, um in dem Bereich des Halses auf den menschlichen Körper aufgebracht zu werden und funktionsfähig um den Hals herum geschlossen zu werden, wobei die Stütze in einem einzelnen Stück gefertigt ist;
- mehr als eine Erkundungssonde (EXP1, EXP2, ......EXPn), extern zu der Stütze, wobei jede Sonde angepasst ist, um Körperparameter zu messen;
- mehr als eine Einheit (U1, U2, .....Un), die angepasst ist, um die Körperparameter zu empfangen, die von einer jeweiligen der Erkundungssonden (EXP1, EXP2, ......EXPn) kommen; wobei jede Einheit angepasst ist, um unabhängig von den anderen Einheiten aktiviert zu werden und wobei die Stütze in der Lage ist, mit einer jeweiligen Einheit zu verbinden;
- eine oder mehrere Verbindungsschnittstellen zum Übertragen und Empfangen von Daten und den Parametern an externe Einheiten;
- elektronische Hardware- und Softwarekomponenten, die im Inneren der flexiblen Stütze integriert sind und angepasst sind, um die Körperparameter von den Einheiten (U1, U2, .....Un) zu empfangen, sie zu speichern, sie zu organisieren und sie auf eine verschlüsselte oder unverschlüsselte Weise mittels der einen oder der mehreren Verbindungsschnittstellen zu übertragen;
- wobei die Vorrichtung angepasst ist, um eine Elektrokardiographie auf eine kontinuierliche oder sofortige Weise aufzuzeichnen und/oder um den Doppler-Ultraschall der aufsteigenden Aorta zu erhalten und/oder um eine m-B-Modus-Echokardiographie zu erhalten und/oder um unmittelbar oder entfernt Notfall-Interventionseinheiten, wie beispielsweise einen temporären transkutanen Schrittmacher und/oder einen Herz-Defibrillator, zu betreiben; **dadurch gekennzeichnet, dass**
- die flexible streifenförmige Stütze (SUP) an den Kanten der längeren Seiten Falze (RB1, RB2) umfasst, die angepasst sind, um eine Schiene zu bilden, um auf eine gleitende und entfernbare Weise die eine oder die mehreren Einheiten (U1, U2, .....Un) und/oder die eine oder die mehreren Verbindungsschnittstellen und/oder die elektronischen Hardware- und Softwarekomponenten einzusetzen.

2. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach Anspruch 1, wobei die elektronischen Komponenten durch Batterien, die in die flexible Stütze integriert sind, betrieben werden, wobei der Kragen Verbindungen zum Wiederaufladen der Batterien (BT) und Mittel zum Identifizieren des Patienten (LI) umfasst.

3. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach Anspruch 2, wobei das Mittel zum Identifizieren des Patienten (LI) eine GPS-Satelliten-Ortungsvorrichtung und eine SIM-Karte umfasst, wobei die Vorrichtung in der Lage ist, als ein Smartphone zu dienen.

4. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach Anspruch 1, wobei der Streifen auf die innere Seite der Stütze zu dem Körper der Person hin gedreht ist, während die gegenüberliegende äußere Seite offen ist.

5. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach Anspruch 1, wobei die eine oder die mehreren Einheiten (U1, U2, .....Un) angepasst sind, um mittels der elektronischen Komponenten miteinander zu kommunizieren.

6. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach einem der vorstehenden Ansprüche, wobei die flexible streifenförmige Stütze ein Kragen ist oder wie eine Kette geformt ist.

7. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach einem der vorstehenden Ansprüche, wobei die medizinische Vorrichtung aus Kunststoff oder Kohlefaser gefertigt ist und angepasst ist, um auf dem menschlichen Körper in einer Position positioniert zu sein, die die kardinale bioelektrische Aktivität nicht beeinträchtigt.

8. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach Anspruch 7, wobei die medizinische Vorrichtung weiter ein Befestigungsmittel umfasst, das an den Enden der flexiblen Stütze platziert ist.

9. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach einem der vorstehenden Ansprüche, wobei die medizinische Vorrichtung eine Stütze für ein Smartphone oder andere Übertragungsmittel umfasst.

10. Medizinische Vorrichtung, angepasst, um auf eine unblutige und nichtinvasive Weise zu erfassen, zu messen und um wichtige biomedizinische Parameter, wie beispielsweise kardiovaskuläre und respiratorische, des menschlichen Körpers zu übertragen, nach Anspruch 1, umfassend Platten, die angepasst sind, um an anatomischen Stellen platziert zu werden und um mit den Erkundungssonden (EXP1, EXP2, ......EXPn) verbunden zu werden, wobei die Platten angepasst sind, um ein Sitz für Datenintegration und-übertragung in einem drahtlosen oder Bluetooth-Modus zu werden.

## Revendications

1. Dispositif médical adapté pour détecter, mesurer, sans saignement et de façon non invasive, et transmettre des paramètres biomédicaux vitaux tels que des paramètres cardiovasculaires et respiratoires du corps humain ; le dispositif comprend :
- un support souple en forme de bande (SUP) adapté pour être appliqué sur le corps humain dans la zone du cou et pour être fonctionnellement fermé autour du cou, ledit support étant fait d'un seul morceau ;
- plusieurs sondes exploratoires (EXP1, EXP2, ..., EXPn) externes au support, chaque sonde étant adaptée pour mesurer des paramètres corporels ;
- plusieurs unités (U1, U2, ..., Un) adaptées pour recevoir lesdits paramètres corporels provenant de chacune desdites sondes exploratoires (EXP1, EXP2, ..., EXPn) ; chaque unité étant adaptée pour être activée indépendamment des autres unités et ledit support pouvant connecter chaque unité ;
- une ou plusieurs interfaces de connexion pour transmettre et recevoir des données et lesdits paramètres vers des unités externes ;
- des composants électroniques matériels et logiciels intégrés à l'intérieur du support souple et adaptés pour recevoir lesdits paramètres corporels desdites unités (U1, U2, ..., Un), les stocker, les organiser et les transmettre sous forme cryptée ou non cryptée au moyen desdites une ou plusieurs interfaces de connexion ;
- ledit dispositif étant adapté pour enregistrer un électrocardiogramme, de façon continue ou instantanée, et/ou pour acquérir l'échographie doppler de l'aorte ascendante, et/ou pour acquérir une échographie cardiaque en mode m-B, et/ou pour fonctionner immédiatement ou à distance sur des unités d'intervention d'urgence telles qu'un stimulateur cardiaque transcutané temporaire et/ou un défibrillateur cardiaque,
**caractérisé en ce que**
- ledit support souple en forme de bande (SUP) comprend des replis (RB1, RB2), au niveau du bord des côtés longs, adaptés pour former un rail pour insérer de façon coulissante et amovible lesdites une ou plusieurs unités (U1, U2, ..., Un), et/ou lesdites une ou plusieurs interfaces de connexion, et/ou lesdits composants électroniques matériels et logiciels.

2. Dispositif médical adapté pour détecter, mesurer de façon non invasive et transmettre des paramètres biomédicaux vitaux tels que des paramètres cardiovasculaires et respiratoires du corps humain selon la revendication 1, dans lequel lesdits composants électroniques sont alimentés par des batteries (BT) intégrées au support souple, ledit col comprenant des connexions pour recharger les batteries et des moyens pour identifier le patient (LI).

3. Dispositif médical adapté pour détecter, mesurer de façon non invasive et transmettre des paramètres biomédicaux vitaux tels que des paramètres cardiovasculaires et respiratoires du corps humain selon la revendication 2, dans lequel lesdits moyens pour identifier le patient (LI) comprennent un localisateur GPS par satellite et une carte SIM, ledit dispositif pouvant servir de smartphone.

4. Dispositif médical adapté pour détecter, mesurer de façon non invasive et transmettre des paramètres biomédicaux vitaux tels que des paramètres cardiovasculaires et respiratoires du corps humain selon la revendication 1, dans lequel ladite bande est tournée sur la face interne du support vers le corps de la personne, tandis que la face externe opposée est exposée.

5. Dispositif médical adapté pour détecter, mesurer de façon non invasive et transmettre des paramètres biomédicaux vitaux tels que des paramètres cardiovasculaires et respiratoires du corps humain selon la revendication 1, dans lequel lesdites une ou plusieurs unités (U1, U2, ..., Un) sont adaptées pour communiquer les unes avec les autres au moyen desdits composants électroniques.

6. Dispositif médical adapté pour détecter, mesurer de façon non invasive et transmettre des paramètres biomédicaux vitaux tels que des paramètres cardiovasculaires et respiratoires du corps humain selon l'une quelconque des revendications précédentes, dans lequel ledit support souple en forme de bande est en forme de col ou de collier.

7. Dispositif médical adapté pour détecter, mesurer de façon non invasive et transmettre des paramètres biomédicaux vitaux tels que des paramètres cardiovasculaires et respiratoires du corps humain selon l'une quelconque des revendications précédentes, dans lequel le dispositif médical est fait de fibres de carbone ou de plastique, adapté pour être positionné sur le corps humain dans une position qui n'interfère pas avec l'activité bioélectrique cardiaque.

8. Dispositif médical adapté pour détecter, mesurer de façon non invasive et transmettre des paramètres biomédicaux vitaux tels que des paramètres cardiovasculaires et respiratoires du corps humain selon la revendication 7, dans lequel le dispositif médical comprend en outre un fermoir qui est placé aux extrémités du support souple.

9. Dispositif médical adapté pour détecter, mesurer de façon non invasive et transmettre des paramètres biomédicaux vitaux tels que des paramètres cardiovasculaires et respiratoires du corps humain selon l'une quelconque des revendications précédentes, dans lequel le dispositif médical comprend un support pour un smartphone ou d'autres moyens de transmission.

10. Dispositif médical adapté pour détecter, mesurer de façon non invasive et transmettre des paramètres biomédicaux vitaux tels que des paramètres cardiovasculaires et respiratoires du corps humain selon la revendication 1, comprenant des plaques adaptées pour être placées sur des sites anatomiques et connectées auxdites sondes exploratoires (EXP1, EXP2, ..., EXPn), lesdites plaques étant adaptées pour devenir le siège de l'intégration des données et de leur transmission en mode sans fil ou Bluetooth.
